# EUROPEAN PATENT SPECIFICATION

(11) **EP 2 167 191 B1**
(45) Date of publication and mention of the grant of the patent: **01.12.2010**
(21) Application number: 08766999.0
(22) Date of filing: 30.05.2008
(51) Int. Cl.: A61N 2/02

(54) **APPARATUS FOR UNDERWATER MUSCLE STIMULATION**
GERÄT ZUR UNTERWASSER-MUSKELSTIMULATION
APPAREIL DE STIMULATION MUSCULAIRE SUBAQUATIQUE

(30) Priority: 03.07.2007 RS 2892007
(43) Date of publication of application: 31.03.2010
(73) Proprietor: Kolpa, d.d. Metlika, 8330 Metlika (SI)
(72) Inventor: Randjelovic, Vladimir, 18000 Nis (RS)
(74) Representative: Pipan, Marjan
(86) International application number: PCT/RS2008/000015
(87) International publication number: WO 2009/005385

(56) References cited:
- EP-A- 0 841 076
- WO-A-01/01919
- WO-A-96/11723
- WO-A-99/25256
- DE-A1- 10 242 542
- DE-A1- 19 824 504
- US-A- 5 047 005

## Description

### Technical Field

Invention is related to the field of magnetic stimulation of neuromuscular tissue in underwater conditions.

### Background Art

Prior art includes a wide variety of solutions for electric and magnetic stimulation of neuromuscular tissue. Since underwater stimulation of muscles has been described little so far, and since electric stimulation would not be convenient for usage in underwater conditions, this invention uses magnetic field for stimulation of muscles located under the water surface, as also taught by WO 99/25256.

Therefore, a system comprising the water vessel (bathtub) equipped with magnetic stimulation unit and sensors, used to provide information about the presence of muscles in the vicinity of the magnetic inductors, has not been described so far.

### Disclosure of Invention

Having in mind the healing effects of water on skeletal and muscular tissue, the invention as defined in claim 1 is designed to generate the contraction of muscles submerged under the surface of water.

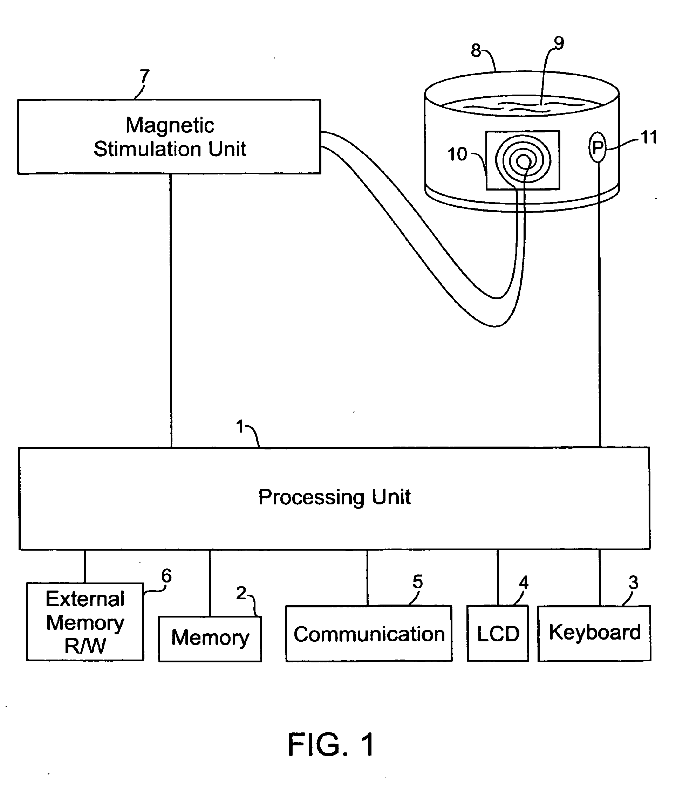

As Fig. 1 shows, the invention comprises water vessel (bathtub, shower booth or similar) (8) equipped with at least one magnetic inductor (10) that is connected to magnetic stimulation unit (7). This unit is used to provide sufficiently large current pulse in a short time through the inductor (10) circuit in order to create high intensity magnetic field capable to produce muscular contraction. Water vessel (8) can be equipped with one or more position sensors (11) used to detect the presence of muscles in the vicinity of inductors and to allow the processing unit (1) to activate stimulation if the user's body is in proper position, or to stop it otherwise.

The whole system is controlled by the processing unit (1) which can also be connected with memory (2), external memory reader/writer (6) used for different types of flash memory cards (SD, MMC), communication system (5) used for connection with other devices, and with user interface comprising keyboard (3) and display (4). All systems connected with the processing unit are explained in further text.

**System for magnetic field stimulation of neuromuscular tissue** (7, 10, Fig. 1) is well explained in a prior art. This system is designed to generate high intensity magnetic pulse with magnetic induction of around IT, during the short time period of around 1ms. This design allows fast change of magnetic flux in user's tissue, which further leads to induction of electric current and neuromuscular stimulation.

System basically consists of magnetic stimulation unit (7) and inductor (10). Magnetic stimulation unit (7) generates current pulse of sufficiently high intensity and sufficiently short rise time in order to produce fast magnetic flux change within the user's neuronal and muscular tissue.

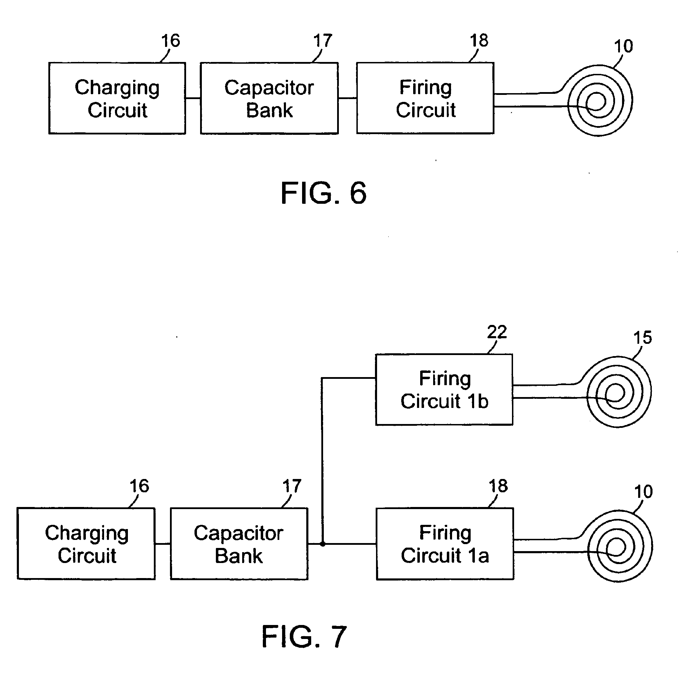

Basic elements of magnetic stimulation unit are shown on Fig. 6. To generate magnetic field of described characteristics, the invention uses the principle of capacitor bank (17) charging over the charging circuit (16) which allows capacitors to be charged to different voltage levels. When voltage level on capacitors achieves defined voltage, charging is stopped. Processing unit is then allowed to send signal to the firing circuit (18) of the magnetic stimulation unit (7, Fig. 1), which discharges the capacitors over the inductor (10) generating the high intensity current pulse through the inductor. High intensity current pulse generates high intensity magnetic field that is capable to stimulate muscle contraction.

The principle of magnetic pulse generation by firing charged capacitors through the inductor is well explained in the prior art and is subject of many patents and patent applications.

Since water vessel (bathtub) can be equipped with several inductors, this invention deals with different ways of connection between inductors and magnetic stimulation units. Basically it recognizes two different types of magnetic stimulation units.

First type of magnetic stimulation unit (Fig. 6) contains one firing circuit (18) used to discharge capacitors (17) through the inductor (10). In this type of magnetic stimulation unit one capacitor bank is discharged over only one inductor.

Second type of magnetic stimulation unit (Fig. 7) contains two or more firing circuits (18, 22) used to discharge one capacitor bank (17) through two or more inductors (10, 15) while each inductor corresponds to one firing circuit.

The number and type of magnetic stimulation units, as well as the number of inductors in the water vessel (bathtub, shower booth or similar) could vary depending on the bathtub model. Two described types of magnetic stimulation units differ with regard to production cost and number of magnetic pulses they are capable to generate in a single inductor. Both types of magnetic field units (Fig. 6, Fig. 7) contain one capacitor bank (17) which is charged over the charging circuit (16). Having in mind that capacitor charging is time consuming process, the first type of the magnetic stimulation unit (Fig. 6) spends time necessary for capacitor charging for the purposes of one inductor only, while the other type of magnetic stimulation unit (Fig. 7) spends the same amount of time for the purposes of two different inductors (10, 15). Because of this, second type of magnetic stimulation unit, equipped with more than one firing circuit (Fig. 7), is capable to generate the same number of charging cycles per unit time as the magnetic stimulation unit with one firing circuit (Fig. 6), but in the latter case, the same number of charging cycles is used for magnetic pulses on two inductors (10, 15, Fig. 7).

On the other hand, magnetic stimulation unit with more than one firing circuit and only one capacitor bank has lower production cost since several inductors use the same capacitor bank and one charging circuit. Because of this, the apparatus presented here can be equipped with one or more inductors and can combine different types of magnetic stimulation units to which inductors are connected.

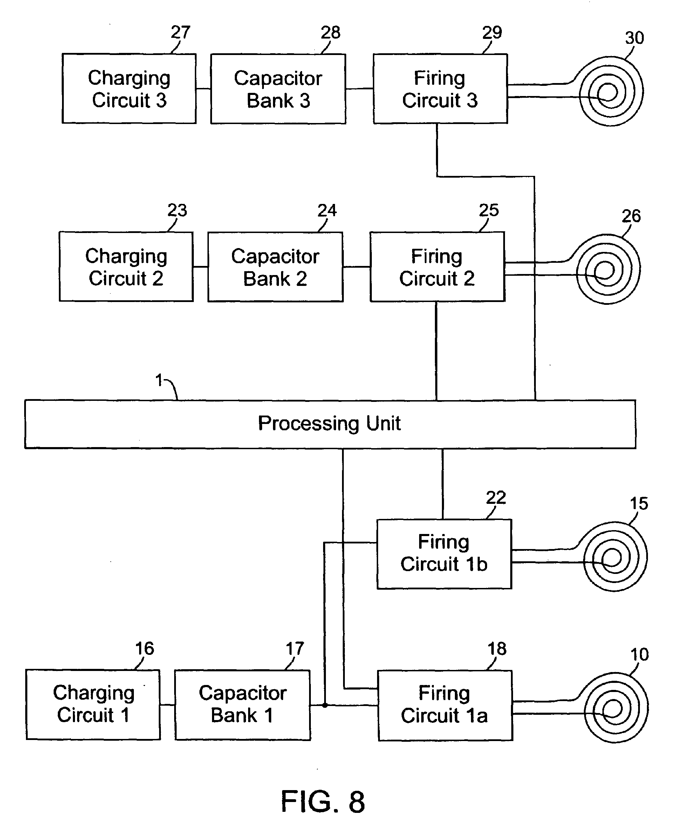

Fig. 8 shows the processing unit (1) connected to one magnetic stimulation unit with two firing circuits (18, 22) which, through inductors (10, 15) fire the charge from one capacitor bank (17). Besides, processing unit is connected with two additional magnetic stimulation units each with one firing circuit (25, 29) over which the charge from capacitor banks (24, 28) is fired through inductors (26, 30). These two capacitor banks are charged over separate charging circuits (23, 27).

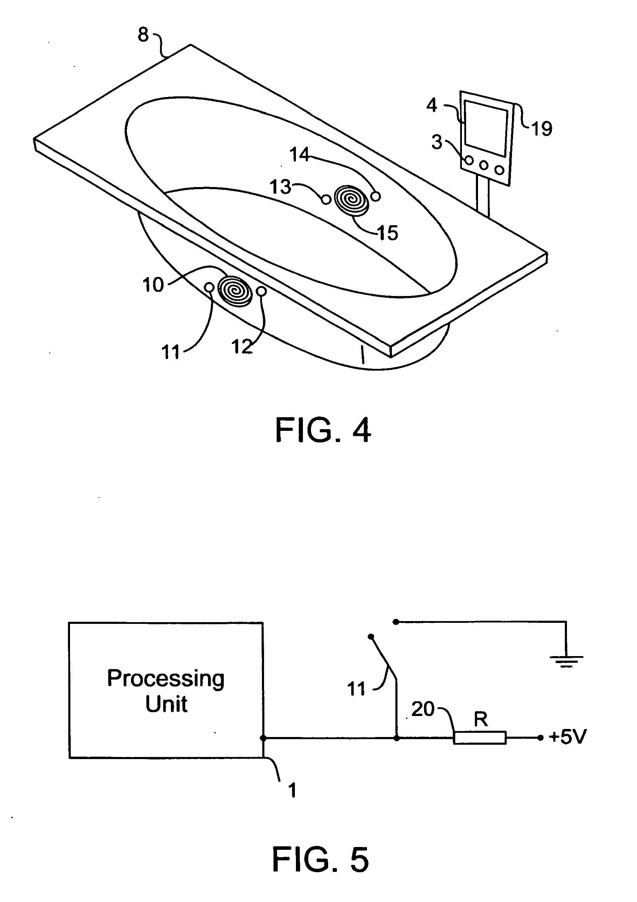

Inductors (10, 15, Fig. 4) for neuromuscular stimulation are well explained in prior art. Several different types of inductors are described and common usage includes circular and butterfly shaped inductors. Inductors usually use large diameter wire with small number of wounds and are characterized by low level of electrical resistance.

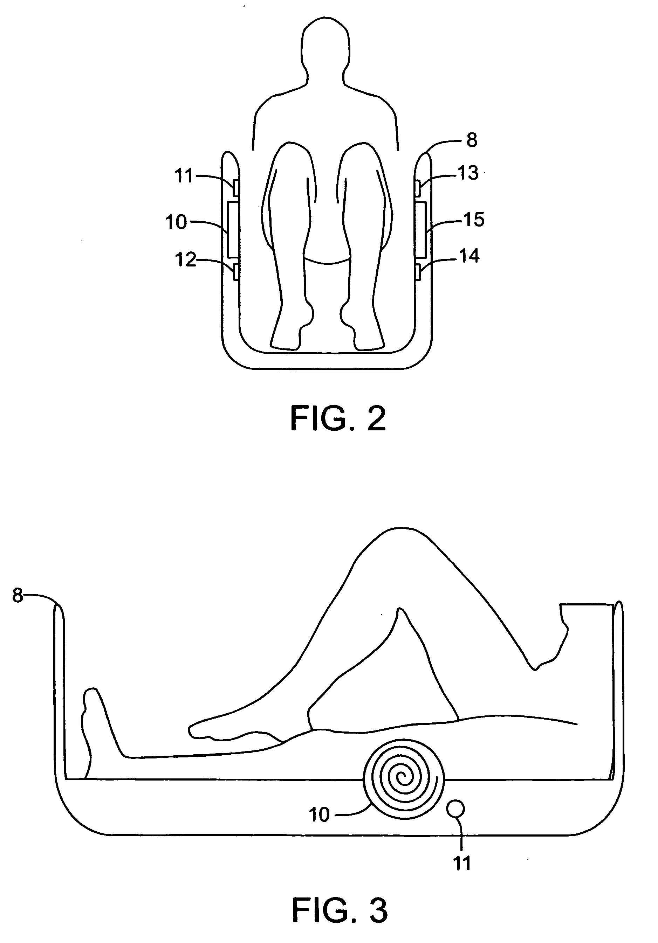

**Water vessel** (8, Fig. 4) is usually used in the form of bathtub or shower booth. Bathtub can be additionally equipped with hydro and air massage systems. Vessel should be designed ergonomically in such way that muscular trigger points of the user ideally fit the position of inductors. Fig. 3 shows bathtub equipped with magnetic inductor (10) the position of which fits the trigger point of one of the user's muscles. This location allows inductor to generate magnetic field at position at which it is possible to produce muscular contraction with lowest possible intensity of the magnetic field.

**Position sensors or switches** (11, 12, 13, 14, Fig. 4) are placed in the vicinity of magnetic inductors in order to detect the presence of user's body. Information about the presence of the user's body is sent to the processing unit. In case the apparatus is activated, processing unit checks the status of position sensors or switches, and in case sensors detect that user's body is in proper position, processing unit initiates magnetic field stimulation. Series of magnetic pulses produce contraction of muscles located close to the inductors.

Prior art includes large number of position sensors that use ultrasound, light, laser, capacitive, inductive or other methods to detect position of the user's body.

Instead of described sensors, ergonomically shaped mechanical switches could be used. Body pressure over the switch (11, Fig. 5) would either open or close the switch circuit and send signal to the processing unit. One of the embodiments of the switch circuit is presented on Fig. 5., where the switch (11) is directly connected to the processing unit. Switch is also connected to the ground potential presenting the logical 0 and, over the pull up resistor (20), to the voltage (+5V) representing the logical 1. In this case, switch pressed by the user's body sends logical 0, while absence of user's body sends logical 1 to the processing unit.

**User interface system** comprises the keyboard (3, Fig. 1) and display (4). Keyboard and display (usually LCD) are connected directly to the processing unit. During the operation of the apparatus, processing unit checks the state of the keyboard and determines which of the buttons is pressed. In case that button is pressed, the software of the processing unit decides about procedures that should be activated. LCD is also connected to the processing unit directly and is updated in certain time intervals.

Communication system (5) with other devices or systems is internally supported by the processing unit. The communication with other devices could be accomplished over the USB, Ethernet, CAN, RS232, RS485, SPI or other types of communication. The communication system allows the connection of the presented apparatus to other devices such as PC, Bathtub controller, mobile phone, audio devices and similar.

USB communication can be accomplished by direct connection of the processing unit (1) with the USB port. SPI communication is accomplished in very short distances by direct connection of the processing unit (1) with processing units of other devices. RS232, RS485, CAN and Ethernet communication require driver integrated circuits witch are, on one side, directly connected to the processing unit (1), while on the other side driver circuits are connected to the corresponding connector.

Besides mentioned communication protocols, communication system (5) supports wireless connection that is accomplished with any RF integrated circuit connected directly to the processing unit (1) on one side, and to antenna on the other side. One of the RF solutions includes usage of Bluetooth integrated circuit which allows radio communication on short distances. In addition to radio communication, processing unit can also be connected to any of the available infrared communication circuits.

Apparatus for underwater muscle stimulation is designed to stimulate neuromuscular tissue and produce muscle contraction using the high intensity magnetic pulse fired in the proximity of the user's muscular trigger point. This pulse is generated within the magnetic stimulation unit (Fig. 6) which, using the firing circuit (18), instantly fires the charge from the capacitor bank (16) through the inductor (10). Powerful current pulse through the inductor wires produces magnetic pulse of high induction which is capable to depolarize neuromuscular cells in the proximity of the inductor.

While lying in the bathtub, user places arms and legs in predefined position, posing the muscles in the proximity of magnetic inductors. Using the keyboard (3) and LCD (4) located on the control panel (19, Fig. 4), user defines the treatment parameters such as number of pulses per minute, treatment time, contraction duration, relaxation duration, and intensity. Besides this, the apparatus allows the user to define treatment parameters using the external memory reader/writer (6, Fig. 1) by putting either CD, DVD, or any type of flash memory cards (SD, MMC).

After setting the treatment parameters, user activates the device by pressing the button on the keyboard. Position sensors/switches (11, 12, 13, 14, Fig. 4) recognize the presence of muscles and activate series of pulses according to the chosen therapy parameters only through inductors close to which user's muscles are detected.

### Brief Description of Drawings

Fig. 1 is a simplified schematic of the invention.
Fig. 2 shows the bathtub with two inductors and four position sensors/switches.
Fig. 3 shows the user lying in a bathtub with inductor located close to the muscular trigger point.
Fig. 4 depicts a bathtub with inductors, position sensors/switches and a control panel.
Fig. 5 is a schematic of position switch circuit.
Fig. 6 shows basic components of magnetic stimulation unit.
Fig. 7 shows components of magnetic stimulation unit with one capacitor bank and two firing circuits.
Fig. 8 shows combined way of inductor connection where each of the two inductors (26, 30) is connected with a separate capacitor bank (24, 28) over the separate firing circuit (25, 29), while the remaining two inductors (10, 15) are connected to one capacitor bank (17) over two separate firing circuits (18, 22).

## Claims

1. Apparatus for underwater muscle stimulation comprising a firing circuit, a capacitor bank and a water container with incorporated inductor connected, over said firing circuit, to said capacitor bank, **characterized in that** said inductor, firing circuit and capacitor bank are designed to generate magnetic pulses with magnetic induction of around 1T during the period of around 1ms, said pulses being capable to stimulate muscle contraction under water within said water container.

## Patentansprüche

1. Apparat für Unterwasser Muskelstimulation, bestehend aus einer Zündschaltung, einer Kondensator-Bank und einen Wasser-Container verbunden mit eingearbeiteten Induktor, über die besagte Zündschaltung, zu der besagten Kondensator-Bank, **gekennzeichnet** in dem besagtem Induktor, Zündschaltung und die Kondensator-Bank sind entworfen, um magnetische Impulse mit magnetischen Induktor von rund um IT im Zeitraum von etwa 1ms zu generieren, besagte Impulse sind fähig Muskelkontraktion unter Wasser innerhalb des besagten Wasser-Container zu stimulieren.

## Revendications

1. L'Appareil pour la stimulation des muscles sous-marine comprenant un circuit de tir, une batterie de condensateurs et un réservoir d'eau avec l'inducteur intégré connecté, sur ledit circuit de tir, notamment la batterie de condensateurs, **caractérisée** dans ce ledit inducteur, le circuit d'allumage et la batterie de condensateurs sont conçus pour générer des impulsions magnétiques à induction magnétique de l'ordre de TI au cours de la période d'environ 1 ms, ces impulsions étant capables de stimuler la contraction des muscles sous l'eau à l'intérieur dudit récipient d'eau.
